(19) **Europäisches Patentamt**

**European Patent Office**

**Office européen des brevets**

(11) **EP 1 001 810 B1**

(12) **EUROPEAN PATENT SPECIFICATION**

(45) Date of publication and mention
of the grant of the patent:
**15.03.2006 Bulletin 2006/11**

(51) Int Cl.:
*A61K 39/395* (2006.01)     *A61K 38/18* (2006.01)

(21) Application number: **98938559.6**

(86) International application number:
**PCT/CA1998/000749**

(22) Date of filing: **05.08.1998**

(87) International publication number:
**WO 1999/007410 (18.02.1999 Gazette 1999/07)**

(54) **PHARMACEUTICAL COMPOSITION WITH NEUROTROPHIC-LIKE BIOLOGICAL ACTIVITY**

PHARMAZEUTISCHE ZUSAMMENSETZUNGEN MIT NEUROTROPHISCHEN BIOLOGISCHEN
AKTIVITÄT

PREPARATION PHARMACEUTIQUE A ACTIVITE BIOLOGIQUE DU TYPE NEUROTROPE

(84) Designated Contracting States:
**AT BE CH CY DE DK ES FI FR GB GR IE IT LI LU
MC NL PT SE**

(30) Priority: **05.08.1997 CA 2212315**

(43) Date of publication of application:
**24.05.2000 Bulletin 2000/21**

(73) Proprietor: **McGill University
Montreal,
Québec H3A 2T5 (CA)**

(72) Inventors:
• **SARAGOVI, H., Uri
Westmount, Quebec H3Z 2L4 (CA)**
• **MALIARTCHOUK, Sergei
Cote Saint-Luc, Quebec H4W 1S8 (CA)**

(74) Representative: **Marks & Clerk
Incorporating Edward Evans Barker
Clifford's Inn
Fetter Lane
London EC4A 1BX (GB)**

(56) References cited:
**WO-A-95/15180**          **WO-A-97/21732**
**US-A- 5 488 099**

• **MALIARTCHOUK S. ET AL: "OPTIMAL NERVE
GROWTH FACTOR TROPHIC SIGNALS
MEDIATED BY SYNERGY OF tRKa AND P75
RECEPTOR-SPECIFIC LIGANDS" THE JOURNAL
OF NEUROSCIENCE, vol. 17, no. 16, 15 August
1997, pages 6031-6037, XP002085192**
• **ZHENG J L ET AL: "NEUROTROPHIN-4/5,
BRAIN-DERIVED NEUROTROPIC FACTOR, AND
NEUROTROPHIN-3 PROMOTE SURVIVAL OF
CULTERED VESTIBULAR GANGLION NEURONS
AND PROTECT THEM AGAINST
NEUROTOXICITY OF OTOTOXINS" JOURNAL OF
NEUROBIOLOGY, vol. 28, no. 3, November 1995,
pages 330-340, XP000674480**
• **CANOSA M. ET AL: "p75NGFR and TrkA
receptors collaborate to rapidly activate
p75NGFR-associated protein kinase" THE EMBO
JOURNAL, vol. 15, no. 13, 1996, pages 3369-3376,
XP002085193**
• **LESAUTEUR L ET AL: "POTENT HUMAN
P140-TRKA AGONISTS DERIVED FROM AN
ANTI-RECEPTOR MONOCLONAL ANTIBODY"
JOURNAL OF NEUROSCIENCE, vol. 16, no. 4, 15
February 1996, pages 1308-1316, XP000645295
cited in the application**
• **CHANDLERT C.E. ET AL: "A monoclonal
antibody modulates the interaction of nerve
growth factor with PC12 cells" THE JOURNAL OF
BIOLOGICAL CHEMISTRY, vol. 259, 1984, pages
6882-6889, XP002085194 cited in the application**

**Description**

## BACKGROUND OF THE INVENTION

(a) Field of the Invention

The invention relates to a pharmaceutical composition having a neurotrophic-like biological activity which comprises a first compound which binds to and/or activates at least one trk neurotrophin receptor, and a second component which binds to and/or activates p75 receptor.

(b) Description of Prior Art

Nerve Growth Factor (NGF) is a 26 kDa dimeric polypeptide which binds two receptors characterized on the basis of their binding affinity. One NGF receptor is a 140 kDa protein (p140 TrkA) with intrinsic tyrosine kinase enzymatic activity. NGF binds TrkA with intermediate affinity ($K_d$ $10^{-10}$ to $10^{-11}$ M). Another receptor is a 75 kDa protein (p75) that is bound by NGF and other neurotrophins such as BDNF with lower affinity ($K_d$ ~$10^{-9}$ M).

Co-expression of TrkA and p75 on the cell surface leads to the formation of a limited number of high affinity NGF binding sites ($K_d$ ~$10^{-12}$ M), that are presumably composed of p75-TrkA heteromers. However, biochemical detection of p75 and TrkA heteromers has not been conclusive.

While expression of TrkA alone is sufficient for cellular responses (Nebreda AR et al. (1991) Science **252**: 558-563; Rovelli G et al. (1993) Proc. Nat. Acad. Sci. (USA) **90**:8717-21), p75 can regulate TrkA-ligand interactions and signal transduction (Hempstead BL et al. (1989) Science **243**:373-375; Verdi JM et al. (1994) Neuron **12**:33-745; Dobrowsky RT et al. (1995) J. Biol. Chem. **270**:22135-42). Moreover, p75 activates its own signaling pathway (Carter BD et al. (1996) Science **272**:542-545; Canossa M et al. (1996) EMBO J. **15**:3369-3376; Cortazzo, MH et al. (1996) J. Neurosci **16**: 3895-3899). It has been suggested that in certain systems ligand-bound p75 receptors may activate apoptotic signals, while in other systems it is unbound p75 receptors that activate apoptosis.

One problem in elucidating the molecular structure of the functional NGF receptor, in determining the individual role of each receptor, and a putative cross-modulation between TrkA and p75 has been the difficulty in obtaining high affinity ligands that completely discriminate between the receptors. Mutant neurotrophins that bind Trk receptors preferentially over p75 function like wild type neurotrophins in biological assays. However, NGF appears to dock onto multiple sites of TrkA, [the IgG-like domain and/or the leucine zipper domain]. Ligand binding to multiple TrkA sites may cause signaling and may lead to p75 immobilization and p75-independent signals.

The Applicants have previously described a monoclonal antibody (mAb) 5C3 that binds a restricted epitope of TrkA with high affinity, and acts as a full agonist (when compared with NGF) on cells that express TrkA but do not express p75 (LeSauteur L et al . (1996) J Neurosci **16**:1308-1316). In the present study, combinations of the TrkA-specific mAb 5C3 and the p75-specific mAb MC192 (Chandler CE et al. (1984) J. Biol. Chem. **259:**6882-6889) were used as ligands to analyze NGF receptor function in functional and biochemical assays. These mAbs maintain high binding affinity irrespective of and unaffected by expression of co-receptors.

US5488099 relates to pharmaceutical compositions comprising neurotrophins having activities of multiple parental neurotrophins.

Maliartchouk S. et al: "Optimal Nerve Growth Factor Trophic Signals Mediated by Synergy of tRKa and P75 Receptor-Specific Ligans" The Journal of Nueorscience, vol. 17, no. 16, 15 August 1997 (1997-08-15), pages 6031-6037, XP002085192 is a publication of the inventors of this application, and relates to the subject matter of this application.

It would be highly desirable to be provided with a pharmaceutical composition having a neurotrophic-like biological activity.

## SUMMARY OF THE INVENTION

One aim of the present invention is to provide a pharmaceutical composition having a neurotrophic-like biological activity.

Surprisingly, and in accordance with the present invention, the data supports the hypothesis that NGF-trophic signals are mediated by TrkA, and that unbound p75 modulates TrkA trophic function. More importantly, the data shows that optimal agonistic ligand mimicry for a multireceptor complex can be achieved by a combination of the natural ligand and an anti-receptor antibody, or by a combination of two antibodies against different receptors. This information will be useful in the design of artificial agonists in multireceptor systems; including neurotrophin receptors.

In accordance with the present invention there is provided a pharmaceutical composition having a neuronal cell survival biological activity which comprises:

a first compound which binds to and/or activates at least one trk neurotrophin receptor, said first compound being selected from the group consisting of a bivalent trk antibody, a monovalent trk antibody, NGF and a NGF mimic, said NGF mimic being selected from the group consisting of C(92-96) and C(92-97) dimer;
and a second component which binds to and/or activates p75 receptor, said second component being an antibody binding to said p75 receptor
in association with a pharmaceutically acceptable carrier;

wherein C(92-96) is an N-acetylated cyclic peptide with the primary sequence YCTDEKQCY, and C(92-97) dimer is a dimer of the cyclic peptide with the primary sequence YCTDEKQACY, C(92-96) and C(92-97) being cyclic by oxidation and intrachain disulphide bonding of their Cys side chains.

The neurotrophic-like biological activity may be that of NGF, NT-3, neurotrophins such as BDNF, CNTF, GDNF, and cytokines.

The trk receptor may be selected from the group consisting of trkA, trkB and trkC.

## BRIEF DESCRIPTION OF THE DRAWINGS

Figs. 1A and 1B illustrate optimal TrkA tyrosine phosphorylation by concomitant binding of p75 and TrkA;

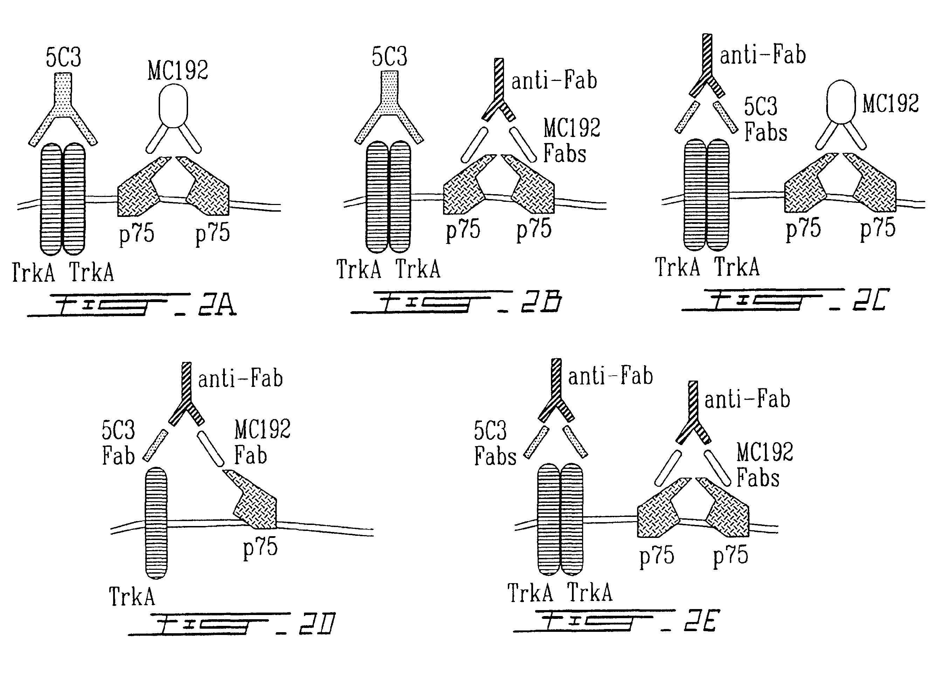

Fig. 2 illustrates various embodiments of the invention.
Fig. 3 illustrates that TrkA binding peptides protect cell from death and that combination of p75 ligands and TrkA binding peptides synergizes and affords nearly 100% protection;
Figs. 4A and 4B illustrate NMR spectroscopy of C(92-96) peptide mimetic; and
Figs. 5A and 5B illustrate that NGF-like signal can be promoted by small molecules and antibodies ligands of TrkA and p75.

## DETAILED DESCRIPTION OF THE INVENTION

Nerve Growth Factor (NGF) receptor-mediated signaling was studied using specific monoclonal antibodies (mAbs) as ligands that discriminate between the receptors TrkA and p75. MAb-induced trophic signals were compared with the signals of the natural ligand NGF. In cells expressing TrkA but no p75 receptors (TrkA$^+$ p75$^-$), binding of TrkA with mAb 5C3 leads to optimal signals. In cells expressing both TrkA and p75 (TrkA$^+$ p75$^+$), binding of TrkA with mAb 5C3 leads to significant but suboptimal signals; and optimal trophic signals are obtained by concomitant binding of TrkA and p75 with mAbs 5C3 and MC192. In TrkA$^+$ p75$^+$ cells binding of anti-p75 mAb MC192 also enhances the trophic effect of suboptimal concentrations of NGF. In contrast, in cells expressing p75 receptors singly (TrkA$^-$ p75$^+$), binding with mAb MC192 or NGF causes very limited or no trophic effects. Thus, the data supports the hypothesis that unbound p75 may modulate TrkA trophic signals. Importantly, the data also demonstrates for the first time that in multireceptor systems appropriate combinations of anti-receptor mAbs can fully mimic the signals of a polypeptide growth factor.

## Cell cultures

Rat PC12 pheochromocytomas cells express p75 and TrkA, B104 rat neuroblastoma cells express ~50,000 surface p75 receptors/cell and none of the Trks (TrkA$^-$ p75$^+$); 4-3.6 cells are B104 cells transfected with human trkA cDNA and express equal levels of surface p75 and TrkA (TrkA$^+$ p75$^+$) . The C10 cell line is a selected subclone of 4-3.6 expressing ~50, 000 surface TrkA receptors but no detectable surface p75 (TrkA$^+$ p75$^-$). Lack of detectable surface p75 receptors on C10 clones was assessed by FACScan analysis (with a sensitivity of <500 receptors/cell). All cell lines were maintained in RPMI media (GIBCO, Toronto, Ontario) supplemented with 5% fetal bovine serum (FBS) and antibiotics. Appropriate drug selection was added to 4-3.6 and C10 cells.

## Antibodies as NGF receptor ligands

Anti-rat p75 mAb MC192 (IgG1; Chandler CE et al. (1984) J. Biol. Chem. **259**:6882-6889) and anti-human TrkA mAb 5C3 (IgG1, LeSauteur L et al. (1996) J Neurosci **16**:1308-1316) ascites were purified with Protein G Sepharose (Pharmacia, Baie d'Urfe, Quebec), dialyzed against phosphate buffered saline and stored at -20;C. MAb 5C3 is agonistic and can fully substitute for NGF in E25 cells expressing TrkA but not p75 (LeSauteur L et al. (1996) J Neurosci **16**: 1308-1316). Further characterization of mAb 5C3 is published in LeSauteur L et al. article ((1996) J Neurosci **16**: 1308-1316). Purified mAbs were characterized by SDS-PAGE under non-reducing or reducing (100 mM 2-mercaptoethanol) conditions to >95% purity.

Binding assays with directly labeled mAbs 5C3 and MC192 demonstrated that each antibody binds to its receptor with relative affinity and saturation profiles irrespective of whether the other receptor is expressed and bound. For example, mAb 5C3 binds similarly to TrkA$^+$ p75$^-$ cells or TrkA$^+$ p75$^+$ cells regardless of whether mAb MC192 is present. This is not unusual or unexpected.

**Protection from cell death**

5,000 cells/well in protein-free media (PFHM-II, GIBCO, Toronto, Ontario) containing 0.1% BSA (Crystalline fraction V, Sigma, St. Louis MO) were added to 96 well plates (Falcon, Mississauga, Ontario). The cultures were untreated or supplemented with serial dilutions of neurotrophins (positive control), test mAbs, or mouse IgG (negative control). The survival profile of the cells was quantitated using the MTT colorimetric assay after 48-72 hours. Percent protection was standardized relative to 1 nM NGF concentrations using the MTT optical density (OD 590 nm) and following formula:

$$[(OD_{test}-OD_{untreated})/(OD\ 1\ nM\ NGF-OD_{untreated})]\ x\ 100.$$

The OD of untreated samples (SFM only) were ~10% of 1 nM NGF control.

Some survival experiments were also carried out in the presence of various concentrations of the tyrosine kinase inhibitor K252a. The concentrations of K252a used were previously reported.

**DNA fragmentation and apoptosis**

Apoptotic death was confirmed by analysis of DNA fragmentation patterns by extraction of genomic DNA. Equal amounts of DNA for each condition were resolved in a 1.5% agarose gel and visualized with ethidium bromide. Note that DNA isolated from apoptotic PC12 cells often does not appear as a typical apoptotic ladder.

**Tyrosine phosphorylation assays**

The tyrosine phosphorylation of TrkA was assayed after a 15 min. treatment of 4-3.6 cells with the indicated agent(s). Analysis was done by Western Blot of whole cell lysates with the enhanced chemoluminescence detection system (ECL, Amersham, Oakville, Ontario) as described (LeSauteur L et al. (1996) J Neurosci **16**:1308-1316) using anti-phosphotyrosine mAb 4G10 (UBI, NY); or affinity purified polyclonal antisera DF-49 recognizing phosphotyrosine PY490 of TrkA which forms the She recognition/docking site on TrkA. Quantitation of protein loading was done with the Bio-Rad Detergent Compatible Protein Assay reagent (Bio-Rad Laboratories), and by coomassie blue staining of gels. Bands in X-ray films were quantified by densitometry [Scanmaster3+ scanner (Howtec Inc., NH) and MSCAN software (Scanalytic, CSP Inc., Hudson, NH)]. Band intensities were standardized using the relative optical density of NGF treatment in each film as 100%. Statistical analysis of densitometry of 3-5 gels were done by paired student t-tests.

**Functional Consequences of NGF Receptor Binding**

Cells undergo apoptotic death when cultured in serum free media (SFM) (Table 1).

**Table 1 p75 binding does not protect from apoptotic death in SFM**

|  | TREATMENT IN SFM CULTURES | PC12 (TrkA$^+$p75$^+$) | B104 (TrkA$^-$p75$^+$) | 4-3.6 (TrkA$^+$p75$^+$) |
|---|---|---|---|---|
| 1 | mouse IgG | 0 ± 2.3 | 0.5 ± 0.7 | 0 ± 2.2 |
| 2 | 1 nM NGF | 100 ± 5.1 | -3 ± 2.2 | 100 ± 4.6 |
| 3 | 100pMNGF | 80 ± 5.5 | 1 ± 1.9 | 87.4 ± 4.5 |
| 4 | 10pMNGF | 40 ± 3.4 | 2 ± 1.7 | 52.3 ± 4.7 |
| 5 | 1 pMNGF | 12 ± 1.1 | -0.5 ± 1.2 | 10.1 ± 5.5 |
| 6 | 2 nM BDNF | not tested | 1 ± 1.3 | 2.1 ± 1.7 |
| 7 | 200 pM BDNF | not tested | 2.5 ± 1.3 | 0.9 ± 1.2 |
| 8 | 20 pM BDNF | not tested | 1.6 ± 1.3 | 3.4 ± 2.2 |
| 9 | 2 pM BDNF | not tested | 0 ± 0.7 | 0.5 ± 3.7 |
| 10 | MC19210nM | 2 ± 1.2 | 0.7 ± 0.8 | 1.2 ± 2.3 |
| 11 | MC1921 nM | -1 ± 3.4 | 0.6 ± 2.3 | 3.7 ± 3.1 |

Table continued

| | TREATMENT IN SFM CULTURES | PC12 (TrkA+p75+) | B104 (TrkA-p75+) | 4-3.6 (TrkA+p75+) |
|---|---|---|---|---|
| 12 | 5 % serum | 157 ± 0.9 | 100 ± 7.7 | 148 ± 7.2 |

PC12, B104, and 4-3.6 cells were cultured in serum free media (SFM), supplemented with test or control ligands as indicated. Cell protection was quantitated after 48 hours by measuring optical density using the MTT colorimetric assay. Data was standardized relative to optimal NGF treatment (PC12 and 4-3.6 cells). B104 cells do not respond to NGF, thus in this assay they were standardized with respect to 5% serum. A representative experiment is shown (average ± standard deviation, n=4) from >3 independent experiments.

B104 cells expressing p75 but not TrkA were not protected by p75 ligands [neurotrophins NGF and BDNF (rows 2-9), or by various concentrations of anti-p75 mAb MC192 (rows 10,11)]. Lack of significant p75 ligand-induced protection in SFM was irrespective of TrkA expression, and apoptotic death occurred in p75+ TrkA+ PC12 cells (Table 1, rows 10,11); and in p75+ TrkA+ 4-3.6 cells (Table 1, rows 6-11). In contrast, NGF binding to TrkA protected cells from apoptotic death in SFM (Table 1, rows 2-5). NGF-mediated protection of PC12 and 4-3.6 cells was dose dependent and consistently suboptimal at ~1-10 pM (Table 1, rows 4 and 5). Standard cell culture conditions containing 5% serum (Table 1, row 12) afford both proliferation and survival. Therefore, higher readings are detected when compared with 1 nM NGF, which in SFM preferentially acts as a survival factor.

Next, cells expressing p75 and human or rat TrkA receptors were used to test potential synergy of mAb MC192 as a p75 ligand and suboptimal NGF doses (5 pM) as a preferential high affinity ligand. MAb MC192 alone affords very limited (or insignificant) protection in SFM (Table 2, rows 4-6; also see Table 1); and 5 pM NGF alone affords suboptimal cell protection ranging from ~30-50% (Table 2, row 3; also see Table 1).

**Table 2 Concomitant p75 and TrkA binding protects cells from apoptotic death**

| | TREATMENT ADDED TO SFM CULTURES | PC12 (TrkA+p75+) | 4-3.6 (TrkA+p75+) |
|---|---|---|---|
| 1 | mouse IgG | 0 ± 1 1 | 0 ± 1.5 |
| 2 | 1 nM NGF | 100 ± 6.5 | 100 ± 4.8 |
| 3 | 5 pM NGF | 28 ± 8.4 | 48 ± 3.5 |
| 4 | MC192 10 nM | 2 ± 1.5 | 1 ± 4.1 |
| 5 | MC192 1 nM | 6 ± 2.7[a] | 6 ± 3.5[a] |
| 6 | MC1920.1 nM | 1 ± 2.0 | 1 ± 4.2 |
| 7 | 5 pM NGF + MC192 10 nM | 49 ± 3.3 | 85 ± 6.5 |
| 8 | 5 pM NGF + MC192 1.0 nM | 86 ± 7.4 | 108 ± 5.6 |
| 9 | 5 pM NGF + MC192 0.1 nM | 26 ± 5.1 | 55 ± 4.5 |
| [a] the small increase in survival induced by mAb MC192 is statistically significant. | | | |

Assays were done as described in Table 1 legend. MAb MC192 synergizes with suboptimal (5 pM) NGF in protecting PC12 and 4-3.6 cells from apoptotic death in SFM (rows 7 and 8).

5 pM NGF + mAb MC192 synergized to significantly increase cell protection in SFM (Table 2, rows 7-9). This protection was dependent on the concentration of mAb MC192, and was maximal at 0.2 mg/ml (1 nM) (Table 2, row 8). MAb MC192 concentrations ranging from 0.1 nM to 1 mM were tested but only some concentrations are shown for clarity. At 2 mg/ml (10 nM) or higher concentrations mAb MC192 afforded limited synergy (Table 2, row 7); and at 0.02 mg/ml (0.1 nM) or lower concentrations it did not synergize with NGF (Table 2, row 9). Thus, a bell-shaped dose response resulted wherein low or high concentrations of mAb do not afford synergy with 5 pM NGF.

Similar tests were performed with 4-3.6 cells (human TrkA+ p75+) and C10 cells (a sorted subclone of 4-3.6 cells, that express human TrkA but is p75-). 4-3.6 and C10 clones express similar number of surface human TrkA receptors. In these cells it is possible to replace NGF with mAb 5C3 as a test ligand for human TrkA (Table 3).

**Table 3 Concomitant ligand binding of p75 and TrkA synergizes in trophic signals**

| | TREATMENT ADDED TO SFM CULTURES | 4-3.6 (TrkA+p75+) | C10 (TrkA+p75-) |
|---|---|---|---|
| 1 | mouse IgG | 0 ± 3.4 | 0 ± 1.7 |
| 2 | 1 nM NGF | 100 ± 5.3 | 100 ± 3.7 |

Table continued

| | TREATMENT ADDED TO SFM CULTURES | 4-3.6 (TrkA$^+$p75$^+$) | C10 (TrkA$^+$p75-) |
|---|---|---|---|
| 3 | 100 pM NGF | 89 ± 6.6 | 35 ± 3.1 |
| 4 | 10 pM NGF | 52 ± 3.6 | 7 ± 3.1 |
| 5 | 1 pM NGF | 4 ± 4.4 | 0 ± 2.4 |
| 6 | 5 nM MC192 | 16 ± 5.7[a] | 1 ± 1.2 |
| 7 | 0.5 nM MC192 | 8 ± 4.0 | 0 ± 1.8 |
| 8 | 5 nM 5C3 | 42 ± 3.0 | 79 ± 5.2 |
| 9 | 0.5 nM 5C3 | 20 ± 5.5 | 64 ± 5.3 |
| 10 | 5 nM 5C3 + 5 nM MC192 | 78 ± 2.7 | 73 ± 3.8[c] |
| 11 | 5 nM 5C3 + 0.5 nM MC192 | 118 ± 3.1[b] | 59 ± 4.9[c] |
| 12 | 0.5 nM 5C3 + 5 nM MC192 | 65 ± 6.8 | 62 ± 1.6[c] |
| 13 | 0.5 nM 5C3 + 0.5 nM MC192 | 96 ± 2.3 | 64 ± 1.4[c] |

[a] the small increase in survival induced by mAb MC192 is statistically significant.

[b] the survival higher than 100% is statistically significant from 1 nM NGF.

[c] not statistically significant from each other.

Assays were done as described in Table 1 legend. Binding of p75 and TrkA with mAbs MC192 and 5C3 respectively (rows 9-12) synergize in protecting 4-3.6 cells from apoptotic death, while binding of TrkA with mAb 5C3 alone (rows 8 and 9) affords suboptimal protection. In contrast, C10 cells are better protected by binding TrkA with mAb 5C3 alone (rows 8 and 9).

Combinations of mAbs 5C3 and MC192 afforded optimal 4-3.6 cell protection (Table 3, row 10-13) which is comparable to that afforded by optimal NGF (Table 3, row 2). Synergy by combination of mAbs 5C3 and MC192 is demonstrated by significantly higher protection than treatment with either mAb alone (Table 3, rows 6-9). Interestingly, while binding of TrkA with mAb 5C3 alone affords only ~20-40% protection to 4-3.6 cells; similar treatment of C10 cells affords 65-80% protection in SFM (Table 3, rows 8 and 9). MAb 5C3 concentrations ranging from 0.01 mg/ml to 5 mg/ml (0.05 nM to 250 nM) were tested but only some concentrations are shown for clarity.

Consistent with C10 cells lacking surface p75, the combination of mAbs MC192 and 5C3 does not enhance the effect of mAb 5C3 alone (Table 3, rows 10-13). Expectedly, C10 cells are less responsive to low doses of NGF than 4-3.6 cells (Table 3, rows 3-5) because they lack detectable p75. Further, no synergy was observed in C10 cells when mAb MC192 and 5 pM NGF were tested in combination.

To assess whether trophic signals leading to cell survival in SFM were mediated via a tyrosine kinase activity, the K252a inhibitor was used (Table 4) .

**Table 4 K252a inhibits NGF receptor-mediated trophic signals**

| K252a (nM) | % CELL SURVIVAL IN SFM SUPPLEMENTED WITH | | |
|---|---|---|---|
| | NGF | 5C3 | 5C3+192 |
| 0 | 100 ± 9 | 50 ± 3 | 112 ± 4 |
| 50 | 60 ± 4 | 32 ± 3 | 67 ± 5 |
| 500 | 32 ± 4 | 13 ± 2 | 43 ± 2 |

Assays were done as described in Table 1 legend. 4-3.6 cell survival in SFM achieved by incubation with the indicated ligands. Optimal ligand concentrations were used as per Table 3 (1 nM NGF, 5 nM 5C3 mAb and 5 nM 5C3 + 0.5 nM MC192 mAbs) . Cells were challenged with various concentrations of K252a and % survival was calculated using 1 nM NGF as 100% standard. K252a inhibits both NGF and mAb-mediated survival in a dose dependent manner and to a similar relative degree.

As expected, K252a inhibited trophic survival induced by 1 nM NGF. K252a also inhibited trophic survival induced by optimal concentrations of mAb 5C3 or by optimal combinations of mAbs 5C3 + MC192. Inhibition by K252a was dose dependent. The highest concentration of K252a tested (500 nM) was not toxic to 4-3.6 cells.

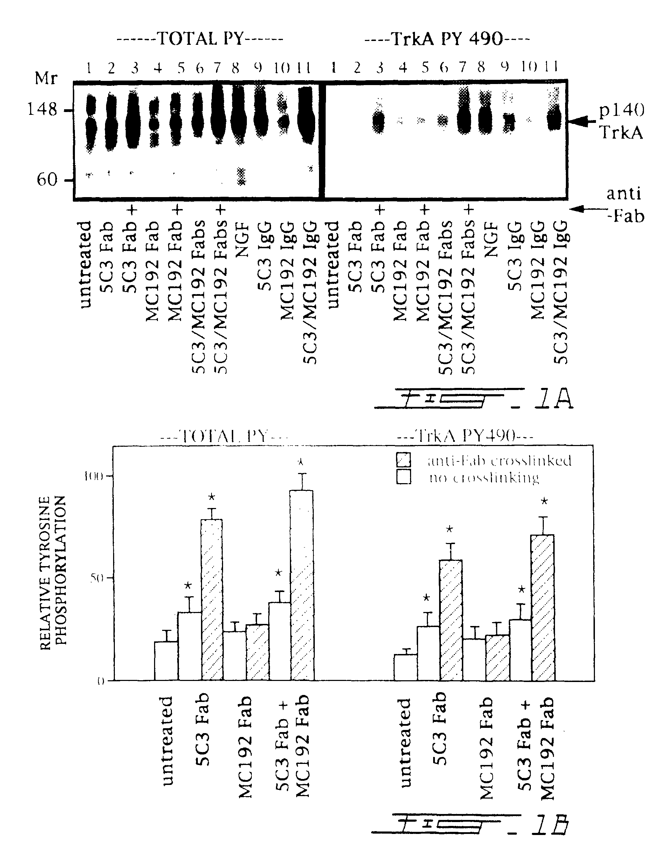

Analysis of the degradation pattern of genomic DNA confirmed the apoptotic nature of cell death in SFM for 4-3.6 and PC12 cells (Fig. 1) and for B-104 cells. Genomic DNA was extracted from (Fig. 1; A) 4-3.6 cells or (Fig. 1; B) PC12 cells cultured as indicated for 48 hours in SFM. Equal amounts from each sample were resolved on a 1.5% agarose gel and visualized with ethidium bromide. Standard molecular markers (M) are shown. A typical apoptotic DNA ladder is seen for 4-3.6 cells, but PC12 DNA is more smeared and difficult to isolate as a ladder. Antibody concentrations were selected from optimal survival assays (e.g. Table 3), namely 5 nM mAb 5C3 and 0.5 nM mAb MC192. 5 pM NGF was suboptimal in survival assays and some DNA laddering is expected (panel B); and that DNA laddering is ablated when NFG is combined with 0.5 nM mAb MC192.

The absence or presence of DNA degradation correlated conclusively with protection or lack of protection from death for all treatments and for all cell lines (Tables 1-3).

In 4-3.6 cells, no DNA degradation is seen upon culture with 5% serum or with mAbs 5C3+MC192; while a small amount of DNA degradation is seen for 4-3.6 cells treated with mAb 5C3 (Fig. 1A). In contrast, extensive apoptotic DNA degradation is seen when 4-3.6 cells are cultured with SFM or mAb MC192 alone (Fig. 1A).

In PC12 cells, no DNA degradation is seen upon culture with 5% serum or with 5 pM NGF+10 nM mAb MC192. PC12 cells treated with 5 pM NGF alone do have limited DNA degradation (Fig. 1B), as expected because this concentration of NGF affords suboptimal survival. PC12 cells cultured with SFM or mAb MC192 alone show extensive DNA degradation (Fig. 1B).

### TrkA tyrosine phosphorylation

To further analyze the signaling mechanism of the antibody-based ligand combinations, TrkA tyrosine phosphorylation (PY) was studied. This was done by western blot analysis of whole cell lysates with antibodies against phosphotyrosine (a-PY), or with antibodies that bind phosphotyrosinylated TrkA within the Shc recognition/docking site [phosphotyrosine 490 of TrkA (a -PY490, DF-49 antibody)].

Initial experiments were designed to resolve the concentration of mAb 5C3 that affords optimal PY of TrkA (Table 5) .

**Table 5 TrkA tyrosine phosphorylation in response to MAb 5C3**

|  |  | C10 CELLS | | 4-3.6 CELLS | |
| --- | --- | --- | --- | --- | --- |
|  | Cells | PYtotal | PY490 | PYtotal | PY490 |
| 1 | no ligand | 11 | 1 | 4 | 1 |
| 2 | NGF 1 pM | 12 | 1 | 7 | 5 |
| 3 | NGF 10 pM | 12 | 1 | 44 | 33 |
| 4 | NGF 100 pM | 36 | 45 | 93 | 61 |
| 5 | NGF 1 nM | 100 | 100 | 100 | 100 |
| 6 | 5C3 0.05 nM | 10 | 1 | 5 | 1 |
| 7 | 5C3 0.5 nM | 40 | 40 | 32 | 21 |
| 8 | 5C3 5 nM | 91 | 71 | 45 | 43 |
| 9 | 5C3 50 nM | 35 | 49 | 39 | 21 |

Cells were untreated (row 1) or treated with the indicated concentrations of NGF (rows 2-5); or mAb 5C3 (rows 6-9); for 15 min at 37°C. Ligand concentrations were selected based on survival assays (e.g. Table 3). Equal amounts of protein from whole cell lysates were resolved by SDS-PAGE and analyzed by western blotting with anti-phosphotyrosine (anti-PY) or with a-PY490 blot (DF-49 sera) recognizing specifically the Shc binding site of TrkA. Band intensities were analyzed by densitometry and standardized using the relative optical density of 1 nM NGF treatment as 100%. Data from a representative western blot is shown.

A 15 minute treatment with mAb 5C3 at 1 mg/ml (5 nM) induced optimal TrkA PY and TrkA PY490 in C10 (TrkA+ p75-) and 4-3.6 cells (TrkA+ p75+). This was consistent with previous survival data (e.g. Table 3). However, 5 nM mAb 5C3 was less efficient at phosphorylating TrkA when compared with 1 nM NGF (Table 5, row 5). This result is also consistent with previous survival data.

As expected, TrkA phosphorylation in response to low NGF concentrations (Table 5, rows 2-4) was decreased in C10 cells compared with 4-3.6 cells, because C10 cells do not express p75 receptors. In contrast, TrkA phosphorylation in response to mAb 5C3 was always stronger in C10 cells compared with 4-3.6 cells (Table 5, row 8).

Using the optimal NGF and mAb 5C3 concentrations above, we studied TrkA PY after treatment of cells with various combinations of the ligands (Fig. 1) 4-3.6 cells were untreated (row 1) or treated with 1 nM NGF(row 2); 5 nM mAb 5C3 alone (row 3) ; 0.5 nM mAb MC192 alone (row 4); or a combination of both mAbs (row 5) for 15 min at 37°C. Ligand concentrations were selected from survival assays (e.g. Table 3) and pilot experiments (e.g. Table 4). Equal amounts of protein from whole cell lysates were resolved by SDS-PAGE and analysed by western blotting.

Fig. 1A- left panel shows an anti-phosphotyrosine (anti-PY) blot. Short thick arrow indicates p140 TrkA. Notable changes in tyrosine phosphorylation of other cellular proteins can be seen induced by NGF, mAb 5C3 or 5C3plus MC192; by NGF or mAb 5C3 only; or by all treatments.

Fig. 1A - right panel shows a -PY490 blot (DF-49 sera) recognizing specifically the Shc binding site of TrkA.

Fig. 1B shows a densitometric scanning quantification of band intensities relative to NGF treatment (average $\pm$ standard error, n=5) . Asterisk (*) indicates significant difference from untreated samples (paired student t-tests, n=5, p<0.03).

A 15 min treatment of 4-3.6 cells (TrkA*) with both 5C3 and MC192 mAbs (Fig. 1A right panel, row 17) induced TrkA PY comparable to that induced by optimal NGF doses (Fig. 1A right panel, row 8) Mab 5C3 alone (Fig. 1A right panel, row 3) caused significant changes in TrkA PY. However, mAb 5C3-induced TrkA PY is lower than that induced by NGF, or by combinations of mAbs 5C3 and MC192. Treatment with mAb MC192 alone did not cause significant changes in TrkA.

Other cellular proteins of sizes ranging from 40-125 kDa are also tyrosine phosphorylated in response to these ligands. Interestingly, the effect upon these unidentified substrates is ligand specific. For example NGF, mAb 5C3, or 5C3+MC192 (but not MC192 alone) cause the PY of a -120 kDa phosphoprotein (Fig 1A left panel); whereas only NGF or mAb 5C3 cause the PY of a ~110 kDa phosphoprotein. All treatments cause the PY of a ~40 kDa phosphoprotein. With the exception of the -40 kDa phosphoprotein, mAb MC192 alone did not cause significant and reproducible increases in PY of other proteins within the 15 minute treatment (Fig. 1A left panel, row 4). More importantly mAb MC192 did not affect TrkA PY in a significant and reproducible manner (Fig. 1A right panel, row 4; see statistical analysis in Fig. 1B).

Densitometry of the TrkA band of five anti-PY blots as in Fig. 1A revealed a significant increase in total PY induced by combination of mAbs 5C3 and MC192 (91% of that induced by optimal NGF) (Fig.1B). The total PY increase induced by treatment with mAb 5C3 alone (56% of that induced by optimal NGF) is significantly higher than untreated control (p=0.029), and it is also significantly different than total PY increases induced by mAB combinations (p=0.022).

Densitometry of the TrkA band of five a -PY490 blots as in Fig. 1A (DF-49 antibody) revealed an increase after treatment with mAb 5C3 (24% of that induced by optimal NGF), which was significantly compared to untreated controls (p=0.016) (Fig. 1B). Treatment with mAbs 5C3+MC192 also increase PY490 (66% of that induced by optimal NGF). The PY490 increases seen after treatment with mAb 5C3, or mAbs 5C3+MC192 are significantly different from each other (p=0.008). Treatment with mAb MC192 alone did not cause a significant increase in TrkA PY490.

Binding of TrkA [with various concentrations of NGF (in PC12 and 4-3.6 cells); or with anti-human TrkA mAb 5C3 (in 4-3,6 cells)] leads to significant trophic signals; as assessed by cell protection in SFM, by increased receptor PY, and by reduced apoptosis and DNA degradation. The signals leading to cell survival in SFM are mediated by a K252a inhibitable tyrosine kinase activity, likely TrkA.

Concomitant binding of TrkA (with the ligands above) and of p75 (with mAb MC192) increase trophic signals synergistically, to levels equivalent to optimal NGF concentrations. When mAbs 5C3 and MC192 are combined there is a small but significant higher 4-3.6 cell survival over optimal NGF. This is likely due to the mAbs being more stable in culture at 37 °C than NGF, and perhaps due to receptor/ligand recycling. The possibility of a small amount of cell division is unlikely because BrdU incorporation in response to mAb 5C3 or NGF in SFM is undetectable.

Synergy of mAb MC192 and NGF in protection frm apoptosis can be explained partially by increased binding of NGF to p75 receptors (Chandler CE et al. (1984) J. Biol. Chem. **259**: 6882-6889. However, several arguments suggest that affinity considerations are not the sole mechanism by which p75 ligands modulate TrkA function first, while NGF increases its affinity for p75 ~3 fold in the present of MC192, the functional enhancement is ~200 fold (survival with 5 pM NGF + MC192 is nearly equivalent to 1 nM NGF). Second, enhancement of p75 affinity by mAb MC192 ought to sequester NGF from TrkA and therefore a *reduction* in TrkA-mediated survival should occur rather than the observed increase. Third and most importantly, mAb MC192 enhances the biological and biochemical function of TrkA stimulated with mAb 5C3. Synergy between these mAb ligands was not due to a change in affinity or binding properties of the mAbs; because each mAb binds its receptor irrespective of and unaffected by the other (see Materials and Methods; Antibodies as NGF receptor ligands).

Functional synergy between p75 ligands and TrkA ligands (in cells expressing both receptors), together with decreased TrkA-mediated signals in TrkA⁺ p75⁺ cells compared with TrkA⁺ p75⁻ cells suggest functional interactions. Two non-exclusive mechanisms may account for the p75 effect: (i) bound p75 positively enhances TrkA signals directly or indirectly; and (ii) unbound p75 negatively modulates TrkA-mediated trophic signals directly or indirectly. Our data provides stronger support for the latter mechanism, based on the following three arguments.

First, decreased trophic signals in response to TrkA binding by mAb 5C3 were detected in 4-3.6 cells (TrkA⁺ p75⁺) when compared with C10 cells (TrkA⁺ p75⁻). Comparable data was published using fibroblasts transfected with trkA cDNA (LeSauteur L et al. (1996) J Neurosci **16**:1308-1316).

Second, synergistic effects occur between TrkA ligands and mAb MC192 only when the concentration of MC192 is optimized to achieve bivalent binding of all or most receptors. At low concentrations (sub-saturating) mAb MC192 does not synergize with TrkA ligands. At very high mAb MC192 concentrations poor synergy is observed, likely because of high dose inhibition (the probability of mAb binding in a monovalent fashion). This is consistent with reports that high doses of mAb MC192 (8 mg/ml; ~40 fold higher than our optimal concentrations) can antagonize the effect of NGF on PC12 cells.

Third, protection from apoptotic death in SFM was very limited or undetectable after binding of p75 alone with NGF (in B104 cells) or with MC192 mAb (in B104; PC12 and 4-3.6 cells); and undetectable after binding with BDNF (in B104 and 4-3.6 cells). The simplest interpretation is that detectable p75 trophic signals in SFM require pre- or co-activation of TrkA. This would be consistent with a possible protein kinase that associates with p75 receptors only after TrkA activation (Canossa M et al. (1996) EMBO J. **15**:3369-3376).

The mechanism by which p75 controls TrkA function probably does not involve TrkA-p75 heterodimers, because they are not likely to be induced by binding of the mAb-based ligands. However, the possibility that receptor heterodimers pre-exist on the cell membrane and are not ligand dependent can not be ruled out. Further, it is also possible that a positive modulation of bound p75 upon TrkA occurs (Canossa M et al. (1996) EMBO J. **15**:3369-3376; Verdi JM et al. (1994) Neuron **12**:33-745).

Previously, polyclonal anti-TrkA antisera was used to achieve ~70% of the neuronal survival afforded by optimal NGF (Clary DO et al. (1994) Mol. Biol. Cell **5**:549-63). The neurons expressed TrkA and p75, but potential synergy upon p75 binding was not studied. Our results are consistent with and expand upon that data.

Although p75 has been reported to signal in the absence of TrkA binding (Carter BD et al. (1996) Science **272: 542-545;** Cortazzo, MH et al. (1996) J. Neurosci **16**: 3895-3899), those p75-mediated signals do not lead to trophic responses or to increased PY of TrkA as studied herein. Our results contrast with other reports wherein unbound p75 receptors did not modulate TrkA-mediated signals (Verdi JM et al. (1994) Neuron **12**:33-745); and p75 binding in the absence of TrkA binding did protect from apoptosis induced by antimitotic agent(Cortazzo, MH et al.(1996) J. Neurosci **16**:3895-3899) . The different results likely are due to the presence of growth factors in these other experiments. Our results also differ to some extent from a report by Rabizadeh et al. (Rabizadeh S et al. (1993) Science **261**:345-348), where p75-mediated TrkA-independent protection from apoptosis was described in NR5D (a line derived from PC12 cells) and CSM14.1 (immortalized neuronal cells), purported to lack TrkA as assessed by Northern blot analysis. However, these cells may express very low levels of TrkA which may help to explain the discrepancy.

Analysis of TrkA PY, particularly the Shc docking site PY490, confirmed that higher activity is induced upon concomitant binding of TrkA and p75. This likely is due to increased kinase kinetics, to lower tyrosine phosphatase activity, or to sustained phosphorylation of PY490. Either of these alternatives support the hypothesis of a negative modulation of TrkA enzymatic activity by unbound p75.

Based on our western blot experiments, the putative negative modulation by p75 seems to be released within a few minutes. Thus, it is unlikely that this modulation involves NFk-b (Carter BD et al. (1996) Science **272**:542-545) or JNK transcriptional pathways. Perhaps the regulation of TrkA by p75 is more direct and acts via phospholipid hydrolysis or other kinases.

Important changes in the PY of cellular proteins other than TrkA are also seen induced by ligands that afford optimal protection from apoptotic death. Some of these proteins are tyrosine phosphorylated in a ligand-specific manner. The identification of these phosphoproteins may reveal differences or specificities in signal transduction induced by NGF versus antibody-based ligands; and will aid in understanding whether the putative negative modulation of TrkA is direct or indirect via adapter or regulatory proteins.

There exist very few anti-receptor mAbs with agonistic activity; and even agonistic polyclonal antisera are rare. Thus, given the dimerizing ability of antibodies it seems that while receptor dimerization is required, it can not alone account for agonistic function. Likely, a conformational change(s) in the structure of the receptor must also occur. We predict that mAb 5C3 affords TrkA homodimerization as well as a partial receptor conformational change(s) that lead to partial agonistic signals.

Partial conformational changes are expected from the fact that mAb 5C3 likely docks onto a region of TrkA and affects the receptor differently than NGF. This is also supported by published observations that mAb monovalent 5C3 Fabs function as agonists in bioassays using fibroblasts transfected with human TrkA (LeSauteur L et al. (1996) J Neurosci **16**:1308-1316). Further, treatment of C10 cells (TrkA[+] p75[-]) with mAb 5C3 affords only -80% of the trophic survival afforded by treatment NGF, suggesting that mAb 5C3 and NGF are not identical TrkA ligands.

Structural analysis of mAb 5C3-TrkA and NGF-TrkA complexes may reveal the nature of the differences, and perhaps putative receptor conformational changes that occur upon ligand binding.

An important and novel concept is the demonstration that functional agonism in a multireceptor system could be optimally achieved by a combination of a natural ligand and an anti-receptor antibody; or by two antibodies against different constituents of the complex. This information might be useful in the design of artificial receptor agonists and antagonists, particularly for neurotrophin or other multireceptor systems.

The present invention will be more readily understood by referring to the following examples.

## EXAMPLE I

**Compounds which binds to NGF receptor TrkA or p75**

PC12 cells were placed in 96-well plates in serum free media as described before. These neuronal-like cells undergo apoptotic death unless they are rescued by ligands of the NGF receptor TrkA or p75.

The indicated ligands were added to the wells at the concentrations shown throughout (NGF either 10 nM or 10 pM; TrkA-binding peptides C(92-96) or C(92-97) at 10 uM; control irrelevant peptide at 10 uM) (the peptides are described in LeSauteur et al., 1996, J. Biol. Chem. **271**:1249); and anti-p75 receptor mAb MC192. After 72 hours cell survival was quantitated with the MTT assay.

Data was standardized to optimal protection by NGF at 1 nM (100%). No treatment or control peptide treatment afforded no protection from death. Optimal NGF treatment afforded 100% survival, and lower doses of 10 pM NGF afforded only ~44% survival.

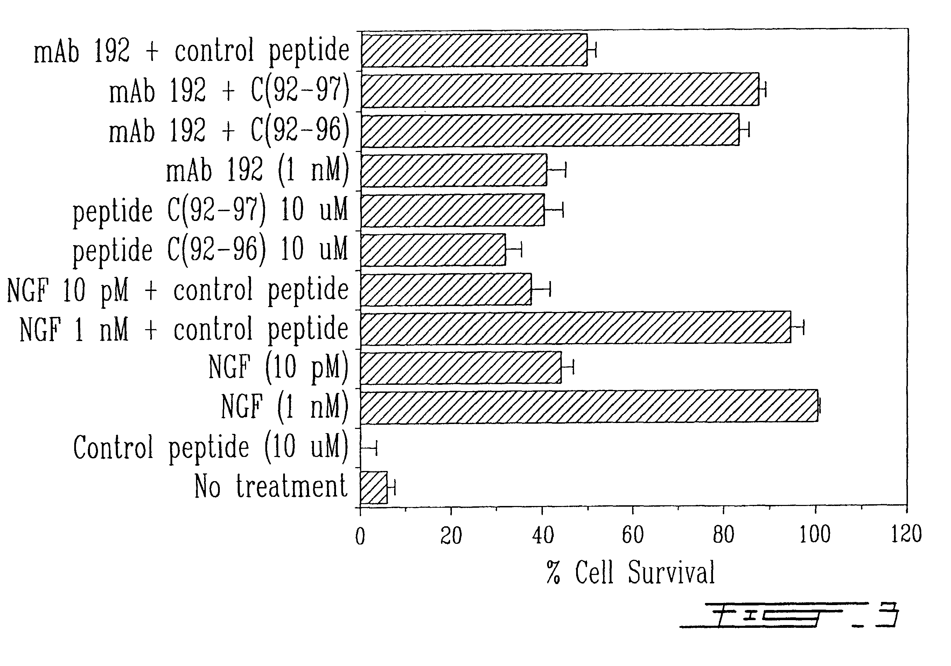

The data demonstrated that the TrkA binding peptides protect cells from death, approaching levels of protection afforded by 10 pM NGF; and that a combination of p75 ligands and TrkA binding peptides synergizes and affords nearly 100% protection (Fig. 3)

A representative experiment is shown, n=8, $\pm$ standard deviation.

## EXAMPLE II

**Small molecules which binds to NGF receptor TrkA or p75**

### Cell culture

PC12 cells are rat pheochromocytomas expressing low levels of rat TrkA and high levels of p75 (TrkA$^+$ p75$^{+++}$). The B104 cells are rat neuroblastomas that express p75 but do not express Trks (TrkA$^-$ p75$^{+++}$). The 4-3.6 cells are B104 cells transfected with human trkA cDNA and express equal surface levels of p75 and TrkA (TrkA$^{+++}$ p75$^{+++}$). The 6-24 cells are PC12 cells transfected with human *trkA* cDNA and overexpressing TrkA (TrkA$^{+++}$ p75$^{+++}$) . Cell surface expression of TrkA and p75 NGF receptors was routinely controlled by FACScan immunofluorescence assays.

### Antibody and fragment preparation

Anti-rat p75 IgG mAb MC192 and anti-human TrkA IgG mAb 5C3 (LeSauteur L et al. (1996) J Neurosci **16**: 1308-1316) were purified from ascites. MAb 5C3 has been described as an agonist of human TrkA, and it does not bind rat TrkA receptors. These antibodies bind to their target irrespective of expression of co-receptors, and regardless of the presence of the other antibody. Purified IgGs were digested with papain (Gibco, Toronto, Ontario) to yield monovalent fragments ($F_{ab}$s). After inactivation of papain, $F_{ab}$s were re-purified with protein G-Sepharose and KappaLock-Sepharose (Upstate Biotechnology, Lake Placid, NY). Intermediate and final products were characterized to >98% purity. No IgG was detected in $F_{ab}$ preparations. Analysis by HPLC and by size exclusion under native conditions did not reveal the presence of aggregates, even at high $F_{ab}$ concentrations. FACScan and other assays demonstrated that $F_{ab}$ preparations bound their receptors in a specific and saturable fashion, indistinguishable from intact IgGs.

### Cyclic NGF mimics

The NGF mimic C(92-96) is an N-acetylated (N-Ac) cyclic peptide with primary sequence Y<u>CTDEKQC</u>Y, and another similar NGF mimic termed C(92-97) has primary sequence Y<u>CTDEKQAC</u>Y. The C(92-96) and C(92-97) peptides are cyclic by oxidation and intrachain disulfide bonding of the Cys side chains (indicated by the underline). The C(92-96) peptide binds TrkA specifically with an apparent $K_d$ ~$10^{-7}$ M (LeSauteur L et al. (1995) J. Biol. Chem. **270**:6564). Linear peptides with the same sequences do not bind TrkA, and were prepared as controls by substituting the Cysteines with Methionines (primary sequence YMTDEKQMY). The linear control analogs can not form disulfide bonds, do not cyclize, and are not conformationally constrained. The C(92-97)$_{dimer}$ is a tethered covalently linked dimer of C(92-97). Analysis by HPLC and mass spectroscopy confirmed the expected retention time and mass for a dimer.

### Peptide synthesis and characterization

N-Ac peptides were synthesized by FMOC chemistry, and purified in a preparative C18 column using reverse phase HPLC. Cyclization, quality control, and characterization of the peptides were done by HPLC and mass spectroscopy (LeSauteur L et al. (1995) J. Biol. Chem. **270**:6564), and by nuclear magnetic resonance (NMR).

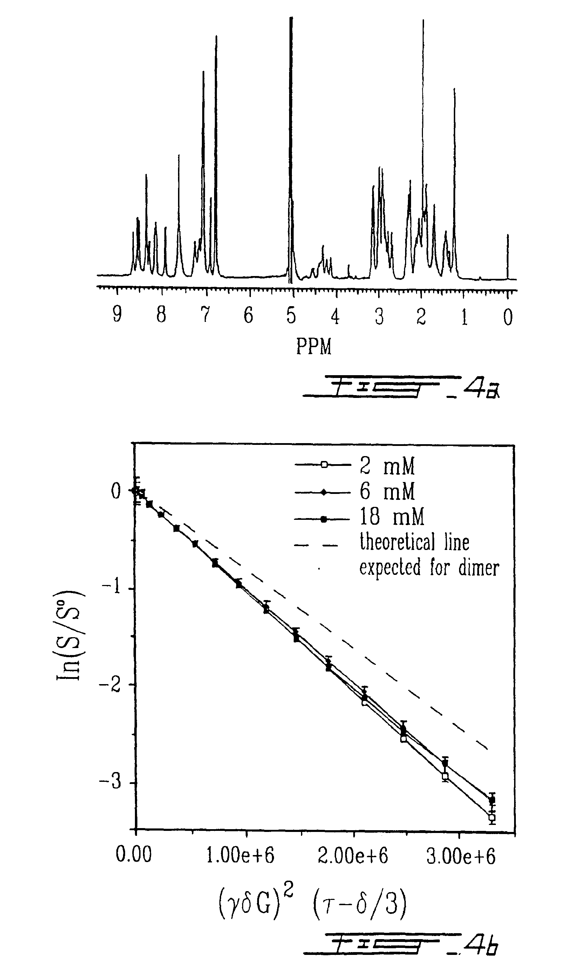

Mass spectroscopy of C(92-96) demonstrated that there were no covalent dimers or oligomers. To determine the aggregation state of the peptide at *millimolar* concentrations *in solution,* high resolution proton NMR spectroscopy was performed at 500 Mhz (Fig. 4A). As will be reported elsewhere, we have determined the solution structure of C(92-96) to better than 0.5 Å root mean square deviation (RMSD) for backbone heavy atoms. NOESY and TOCSY spectra were consistent with a monomeric, unaggregated state. The molecular correlation time of C(92-96) was assessed. Natural $^{13}$C abundance NMR relaxation parameters were measured for the alpha carbon atoms. Fitting the Ca, T1, T2 and heteronuclear NOEs values using a model-free formalism produced an overall correlation time of 1.76 nanoseconds at 5°C as expected for a monomer.

A better criterion for the aggregation state of a peptide is its self-diffusion constant in solution. Translational self-diffusion of C (92-96) is dependent on the hydrodynamic properties and the size of the molecule. Isotropic diffusion measurements unequivocally identified C(92-96) as monomeric. Pulse field gradient NMR measurements of the self-diffusion coefficient (D) were determined at various peptide concentrations of 2.5, 6, or 18 mM; T=278°K. Values of D= $1.01 \pm 0.07$ ($10^{-6}$ cm$^2$/s); D= $1.00 \pm 0.06$ ($10^{-6}$ cm$^2$/s); and D= $1.04 \pm 0.05$ ($10^{-6}$ cm$^2$/s) were measured for 18 mM, 6 mM and 2.5 mM samples respectively (Fig. 4B).

These D values are essentially the same, indicating an identical state for the peptide. Thus, the samples remain monomeric, and peptide aggregates are undetectable in solution even at concentrations as high as 18 mM. We estimate that the self-association constant for any putative aggregate cannot be larger than 10 M$^{-1}$. Thus, a 10 mM solution of C (92-96) could not contain >1 nM self-aggregated dimers, if any aggregate at all.

## *Small molecule monovalent and bivalent partial agonists of TrkA*

The genuine monovalent and monomeric TrkA ligand C(92-96) was tested for trophic support in SFM. To gauge the potency and efficacy of monomeric versus dimeric small molecule TrkA ligands a covalent dimeric analog of C(92-96) termed C(92-97)$_{dimer}$ was evaluated. Furthermore, to study whether TrkA receptor density influences trophic signals mediated by the NGF mimics, the ligands were assayed in parallel on cell lines that only differ in TrkA density (PC12 TrkA$^+$ p75$^{+++}$; versus 6-24 TrkA$^{+++}$ p75$^{+++}$).

### Table 6 Concomitant p75 and TrkA binding protects cells from apoptotic death

|  | TREATMENT | % protection | |
|---|---|---|---|
|  |  | PC12 | 6-24 |
| 1 | 1 nM NGF | $100 \pm 1.8$ | $100 \pm 3.7$ |
| 2 | NGF 10 pM | $30.4 \pm 3.6$ | $59.8 \pm 3.1$ |
| 3 | C(92-96) 10 $\mu$M | $0 \pm 2.7$ | $0.6 \pm 3.1$ |
| 4 | C(92-97)dimer 10 $\mu$M | $5.3 \pm 1.6$ | $23.1 \pm 2.4$ |
| 5 | linear peptide 10 $\mu$M | $1.3 \pm 1.3$ | $8.28 \pm 1.2$ |
| 6 | MC192 IgG 1 nM | $3.6 \pm 2.5$ | $7.38 \pm 1.8$ |
| 7 | C(92-96) + MC192 | $28.0 \pm 2.8$ | $51.7 \pm 5.2$ |
| 8 | linear peptide + MC192 | $5.2 \pm 2.0$ | $7.0 \pm 3.8$ |

The trophic response to 1 nM NGF was optimal in both cell types (Table 6, row 1). However, survival to 10 pM NGF was better in 6-24 cells than in PC12 cells (Table 6 row 2). This suggests that TrkA density influences ligand-induced signals, and high TrkA expression affords better potency when ligand concentrations are limiting.

The C(92-96) NGF mimic did not afford significant trophic support of PC12 or 6-24 cells; compared to control linear peptides (Table 6, rows 3 versus 5). In contrast, the C (92-97)$_{dimer}$ peptide afforded good trophic support for 6-24 cells, and very low but statistically significant support for PC12 cells (Table 6, row 4).

MAb MC192 IgG alone afforded very low or insignificant trophic support (Table 6, row 6); but as a bivalent p75 ligand it can synergize with TrkA ligands (e.g. see Tables 2 and 3). Bivalent mAb MC192 synergized with the monomeric C(92-96) TrkA ligand in protecting 6-24 cells and PC12 cells from apoptotic death in SFM (Table 6, row 7). The combination of C(92-96) plus mAb MC192 was more potent in protecting 6-24 cells. However, the efficacy of this combination was similar for both cell types, and comparable in each case to 10 pM NGF. Bivalent mAb MC192 did not synergize with control linear peptides (Table 6, row 8).

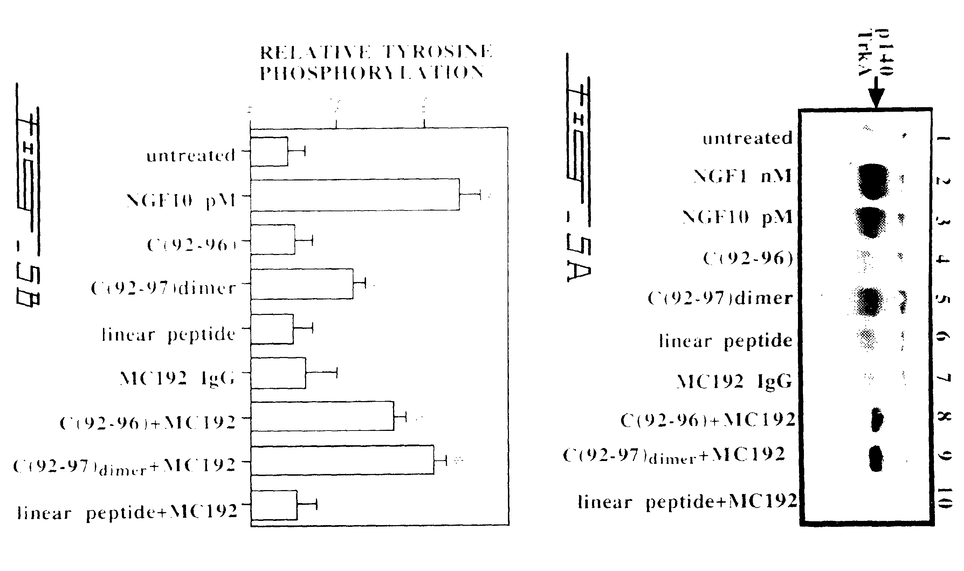

Experiments using monomeric C(92-96) and C(92-97)$_{dimer}$ peptides in 4-3.6 cells resulted in nearly identical data to that obtained in 6-24 cells (see Fig. 5). This was expected because 4-3.6 and 6-24 cells express equivalent TrkA receptor densities, and indicate that the effect is not limited to a cell line. In contrast, controls using B104 cells (TrkA-, p75$^{+++}$ parental to 4-3.6) demonstrated that no protection was afforded by the peptide NGF mimics, alone or in combination with mAb MC192. These data suggest that the bioactivity of the NGF mimics requires TrkA expression.

The concentration at which the peptides C(92-96) and C(92-97)$_{dimer}$ were effective is ~2 orders of magnitude over their apparent $K_d$; at lower concentrations the activity was notably reduced. However, the bioassays compare small molecule NGF mimics *versus* 1 nM NGF which is a concentration of NGF -3 orders of magnitude over its apparent $K_d$. It is possible that the ligands have short half lives in solution at 37°C in bioassays lasting 48-72 hours, or that the ligands are bound by matrix proteoglycans or carrier proteins.

### *Small molecule ligands and TrkA tyrosine phosphorylation*

To further assess whether the trophic support by small cyclic peptides was indeed mediated by TrkA, tyrosine phosphorylation of the receptor was studied in 4-3.6 cells (Fig. 5). Representative anti-PY western blots are shown in Fig. 5A. A summary of densitometric scanning analysis from several blots is given in Fig. 5B.

Treatment with C(92-96) peptide alone did not induce an increase in TrkA PY (Fig. 5A, lane 4) compared with control untreated cells (Fig. 5A, lane 1), control linear peptide treated cells (Fig. 5A, lane 6), or bivalent mAb MC192 treated cells (Fig. 5A, lane 7) . Significant TrkA PY was induced by treatment with C(92-97)$_{dimer}$ (Fig. 5A, lane 5). TrkA PY induced by C(92-97)$_{dimer}$ represented ~29% of that induced by 1 nM NGF (Fig. 5A, lane 2).

Combinations of mAb MC192 and C(92-96) peptide (Fig. 5A, lane 8), or mAb MC192 and C(92-97)$_{dimer}$ peptide (Fig. 5A, lane 9) afforded notable increases in TrkA PY, respectively 41% and 53% of 1 nM NGF. These TrkA PY signals are comparable to those induced by 10 pM NGF (Fig. 5A, lane 3). In contrast, treatment with a combination of bivalent mAb MC192 and linear peptide controls (Fig. 5A, lane 10) did not result in significant increases in TrkA PY. For statistical analysis see Fig. 5B.

### *Monovalent partial agonists of TrkA*

Monovalent TrkA ligands 5C3 F$_{ab}$s and C(92-96) can be partial agonists in trophic assays. Protection from apoptosis by 5C3 F$_{ab}$s was obtained with several cell lines, suggesting that the effect is not cell line specific.

Functional responses to monomeric ligands of TrkA are evident. For example, the genuine monomeric small molecule C(92-96) afforded trophic support under certain conditions, although the bivalent C(92-97)$_{dimer}$ peptide was more efficient. Since the covalent dimeric C(92-97)$_{dimer}$ did not afford trophic responses at nM concentrations, it is unlikely that any C(92-96) self-aggregation in solution beyond the detection of NMR can account for the effects of this monomeric peptide.

It is also highly unlikely that the monomeric C(92-96) is not monovalent because it is too small to interact with two receptors simultaneously. It is well-established that receptor-ligand interactions generally involve contact by 4-30 side chains, and C(92-96) has 5 residues or less in the pharmacophore. Nevertheless, the intriguing possibility that the C(92-96) peptide could dimerize after docking remains unexplored. An answer to this question requires structural analysis of the receptor-ligand complex.

### *Monovalent and bivalent ligands of p75*

Monovalent or bivalent p75 ligands alone do not substantially protect cells from apoptosis; and when monovalent p75 ligands are combined with TrkA ligands the trophic effect of the latter are not potentiated synergistically. However, bivalent p75 ligands do potentiate the effects of TrkA ligands (either mAb 5C3, peptidic NGF mimics, or suboptimal doses of NGF). It is possible that other p75 ligands not tested here may have different effects or synergy.

### Claims

1. A pharmaceutical composition having a neuronal cell survival biological activity which comprises:

   a first compound which binds to and/or activates at least one trk neurotrophin receptor, said first compound being selected from the group consisting of a bivalent trk antibody, a monovalent trk antibody, NGF and a NGF mimic, said NGF mimic being selected from the group consisting of C(92-96) and C(92-97) dimer; and
   a second component which binds to and/or activates p75 receptor, said second component being an antibody binding to said p75 receptor;
   in association with a pharmaceutically acceptable carrier,

   wherein C(92-96) is an N-acctylated cyclic peptide with the primary sequence YCTDEKQCY, and C(92-97) dimer is a dimer of the cyclic peptide with the primary sequence YCTDEKQACY, C(92-96) and C(92-97) being cyclic by oxidation and intrachain disulphide bonding of their Cys side chains.

2. The pharmaceutical composition of claim 1,wherein said antibody binding to said p75 receptor is MC192.

3. The pharmaceutical composition of claim 1 or 2, wherein said bivalent trk antibody is 5C3.

4. The pharmaceutical composition of claim 1 or 2, wherein said monovalent antibody is 5C3 $F_{ab}$s.

5. The pharmaceutical composition of any preceding claim, wherein said trk neurotrophin receptor is selected from the group consisting of A, B, C.

**Revendications**

1. Une composition pharmaceutique ayant une activité biologique de survie de cellule neuronale comprenant :

un premier composé qui est en liaison avec et/ou qui active au moins un récepteur de neurotrophine trk, ledit composé étant choisi parmi le groupe constitué d'un anticorps trk divalent, un anticorps trk monovalent, du NGF et du NGF mimétique, ledit NGF mimétique étant choisi parmi le groupe constitué du C(92-96) et du C(92-97) dimère, et
un second composé qui est en liaison avec et/ou qui active un récepteur p75, ledit second composé étant un antibiotique se liant avec ledit récepteur p75;
en association avec un véhicule pharmaceutique acceptable,

dans laquelle le C(92-96) est un peptide cyclique N-acétylé dont la séquence primaire est YCTDEKQCY, et le dimère C(92-97) est un dimère du peptide cyclique dont la séquence primaire est YCTDEKQACY, les C(92-96) et C(92-97) étant rendus cycliques par oxydation et liaison disulfide intrachaîne de leurs chaînes latérales Cys.

2. La composition pharmaceutique de la revendication 1, dans laquelle l'anticorps se liant avec ledit récepteur p75 est le MC192.

3. La composition pharmaceutique de la revendication 1 ou 2, dans laquelle l'anticorps est le 5C3.

4. La composition pharmaceutique de la revendication 1 ou 2, dans laquelle l'anticorps est le 5C3 $F_{abS}$.

5. La composition pharmaceutique de l'une quelconque des revendications précédentes, dans laquelle ledit récepteur neurotrophine trk est choisi parmi le groupe constitué de A, B, C.

**Patentansprüche**

1. Pharmazeutische Zusammensetzung mit einer biologischen Aktivität auf das Überleben von Nervenzellen, umfassend:

eine erste Verbindung, die an mindestens einen trk-Neurotrophinrezeptor bindet und/oder diesen aktiviert, wobei die erste Verbindung ausgewählt ist aus der Gruppe bestehend aus einem bivalenten trk-Antikörper, einem monovalenten trk-Antikörper, NGF und einem NGF-Mimetikum, wobei das NGF-Mimetikum ausgewählt ist aus der Gruppe bestehend aus C(92-96) und einem C(92-97)-Dimer; und
einen zweiten Bestandteil, der an den p75-Rezeptor bindet und/oder diesen aktiviert, wobei der zweite Bestandteil ein Antikörper ist, der an den p75-Rezeptor bindet;
in Verbindung mit einem pharmazeutisch annehmbaren Träger, wobei C(92-96) ein N-acetyliertes cyclisches Peptid mit der Primärsequenz YCTDEKQCY und das C(92-97)-Dimer ein Dimer des cyclischen Peptids mit der Primärsequenz YCTDEKQACY ist, wobei C(92-96) und C(92-97) durch Oxidation und Disulfidbrückenbindung innerhalb der Kette ihrer Cys-Seitenketten cyclisch ist.

2. Pharmazeutische Zusammensetzung nach Anspruch 1, wobei der Antikörper, der an den p75-Rezeptor bindet, MC192 ist.

3. Pharmazeutische Zusammensetzung nach Anspruch 1 oder 2, wobei der bivalente trk-Antikörper 5C3 ist.

4. Pharmazeutische Zusammensetzung nach Anspruch 1 oder 2, wobei der monovalente Antikörper 5C3 $F_{abS}$ ist.

5. Pharmazeutische Zusammensetzung nach einem der vorhergehenden Ansprüche, wobei der trk-Neurotrophinrezeptor ausgewählt ist aus der Gruppe bestehend aus A, B, C.

FIG. 1A

FIG. 1B

FIG. 2A

FIG. 2B

FIG. 2C

FIG. 2D

FIG. 2E

FIG. 3

% Cell Survival

mAb 192 + control peptide
mAb 192 + C(92-97)
mAb 192 + C(92-96)
mAb 192 (1 nM)
peptide C(92-97) 10 uM
peptide C(92-96) 10 uM
NGF 10 pM + control peptide
NGF 1 nM + control peptide
NGF (10 pM)
NGF (1 nM)
Control peptide (10 uM)
No treatment

_FIG._ _4a_

$(\gamma \delta G)^2 (\tau - \delta/3)$

_FIG._ _4b_

_FIG._5A

_FIG._5B